# EUROPEAN PATENT APPLICATION

(11) **EP 4 566 530 A1**
(43) Date of publication of application: **11.06.2025**
(21) Application number: 24157287.4
(22) Date of filing: 13.02.2024
(51) Int. Cl.: A61B 5/0537, A61B 5/251, A61B 5/00

(54) **CLAMP ELECTRODE DEVICE AND ELASTIC MEMBER FOR THE SAME**

(30) Priority: 05.12.2023 KR 20230174228
(71) Applicant: InBody Co., Ltd., Seoul 06106 (KR)
(72) Inventor: Cha, Ki Chul, 06544 Seoul (KR); Keum, Dong Hoon, 04999 Seoul (KR); Cha, Young Tae, 07522 Seoul (KR)
(74) Representative: Canzler & Bergmeier Patentanwälte Partnerschaft mbB

(57) **Abstract**

A clamp electrode device includes a clamp body including a first clamp wing, a second clamp wing facing the first clamp wing, and a hinge unit supporting an end of each of the first clamp wing and the second clamp wing, an electrode unit including a first electrode on a surface facing the second clamp wing in the first clamp wing and a second electrode on a surface facing the first clamp wing in the second clamp wing, and an elastic member connecting the first electrode to the first clamp wing.

## Description

### BACKGROUND

### 1. Field

The following description relates to a clamp electrode device and an elastic member for the clamp electrode device.

### 2. Description of Related Art

Body composition analysis generally refers to measuring and analyzing a ratio of the water, fat, bone, or muscle of a body of a measurement target, and the information from the analyzed body composition is useful in various fields.

For example, in the fitness field, body composition information may be used to plan exercises or analyze progress or results. Alternatively, in the medical field, body composition information may be used to analyze a body composition state of a patient and treat the patient. The method of measuring body composition includes a method of measuring an electrical impedance value of a body.

### SUMMARY

An aspect provides technology for improving the measurement accuracy or skin adhesion of a clamp electrode device and the convenience of a person wearing the clamp electrode device for examination.

Another aspect also provides technology for relieving pain felt by a subject from the clamp electrode device through a transformable elastic member suitable for various body shapes of the subj ect.

Another aspect also provides technology for overcoming the movement or deviation of an electrode during measurement through an elastic member by considering the characteristics of the clamp electrode device which measures body composition at a fixed position for a certain time (e.g., 1 minute) or more.

However, technical aspects of various embodiments herein are not limited to the foregoing aspects, and there may be other technical aspects.

According to an aspect, there is provided a clamp electrode device including: a clamp body including a first clamp wing, a second clamp wing facing the first clamp wing, and a hinge unit supporting an end of each of the first clamp wing and the second clamp wing; an electrode unit including a first electrode on a surface facing the second clamp wing in the first clamp wing and a second electrode on a surface facing the first clamp wing in the second clamp wing; and an elastic member connecting the first electrode to the first clamp wing.

According to another aspect, there is provided an elastic member for a clamp electrode device including: a support surface facing a fastening target of the clamp electrode device; a sidewall extending from the support surface; and a fastening region that is at an end opposite to the support surface in the sidewall and coupled to the clamp electrode device.

According to an aspect, in a clamp electrode device and an elastic member for the clamp electrode device, a second point of a sidewall forms a relatively small transformable range compared to a first point, which may improve the adhesion between the clamp electrode device and a subject, measurement accuracy, and the convenience of use.

According to an aspect, the clamp electrode device and the elastic member for the clamp electrode device may adjust a transforming shape and transformable range of the elastic member through various structural designs, and the clamp electrode device may be worn by a subject easily and efficiently according to the body conditions of various subjects.

The effects of the clamp electrode device and the elastic member for the clamp electrode device according to an embodiment may not be limited to the above-mentioned effects, and other unmentioned effects may be clearly understood from the following description by one of ordinary skill in the art.

Additional aspects of example embodiments will be set forth in part in the description which follows and, in part, will be apparent from the description or may be learned by practice of the disclosure.

### BRIEF DESCRIPTION OF THE DRAWINGS

These and/or other aspects, features, and advantages of the present disclosure will become apparent and more readily appreciated from the following description of example embodiments, taken in conjunction with the accompanying drawings of which:
FIG. 1 is a conceptual diagram illustrating body composition measurement according to an embodiment;
FIG. 2A is an enlarged view of a part in which an electrode is attached to an arm of a subject in the conceptual diagram of FIG. 1;
FIG. 2B is an enlarged view of a part in which an electrode is attached to a leg of the subject in the conceptual diagram of FIG. 1;
FIG. 3A is a perspective view of a state in which a clamp electrode device is worn on an arm of a subject, according to an embodiment;
FIG. 3B is a perspective view of a state in which the clamp electrode device is worn on a leg of the subject, according to an embodiment;
FIG. 4 is a perspective view of a clamp electrode device according to an embodiment;
FIG. 5 is a cross-sectional view of the clamp electrode device according to an embodiment;
FIG. 6 is a perspective view of an elastic member according to an embodiment;
FIG. 7 is a side view of the elastic member according to an embodiment;
FIG. 8 is another perspective view of the elastic member according to an embodiment;
FIG. 9 is an enlarged view of the elastic member according to an embodiment;
FIG. 10 is another perspective view of the elastic member according to an embodiment;
FIG. 11 is another perspective view of the elastic member according to an embodiment;
FIG. 12 is another perspective view of the elastic member according to an embodiment;
FIG. 13 is a perspective view of a partial configuration of the clamp electrode device according to an embodiment;
FIG. 14A is a perspective view of an elastic member according to an embodiment;
FIG. 14B is a side view of the elastic member according to an embodiment;
FIG. 14C is another perspective view of the elastic member according to an embodiment;
FIG. 14D is an enlarged view of the elastic member according to an embodiment;
FIG. 14E is another perspective view of the elastic member according to an embodiment; and
FIG. 14F is another perspective view of the elastic member according to an embodiment.

### DETAILED DESCRIPTION

The following detailed structural or functional description is provided as an example only and various alterations and modifications may be made to embodiments. Here, examples are not construed as limited to the disclosure and should be understood to include all changes, equivalents, and replacements within the idea and the technical scope of the disclosure.

Terms, such as first, second, and the like, may be used herein to describe various components. Each of these terminologies is not used to define an essence, order or sequence of a corresponding component but used merely to distinguish the corresponding component from other component(s). For example, a first component may be referred to as a second component, and similarly the second component may also be referred to as the first component.

It should be noted that if it is described that one component is "connected", "coupled", or "joined" to another component, a third component may be "connected", "coupled", and "joined" between the first and second components, although the first component may be directly connected, coupled, or joined to the second component.

The singular forms "a", "an", and "the" are intended to include the plural forms as well, unless the context clearly indicates otherwise. It will be further understood that the terms "comprises/including" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art and are not to be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like elements and a repeated description related thereto will be omitted.

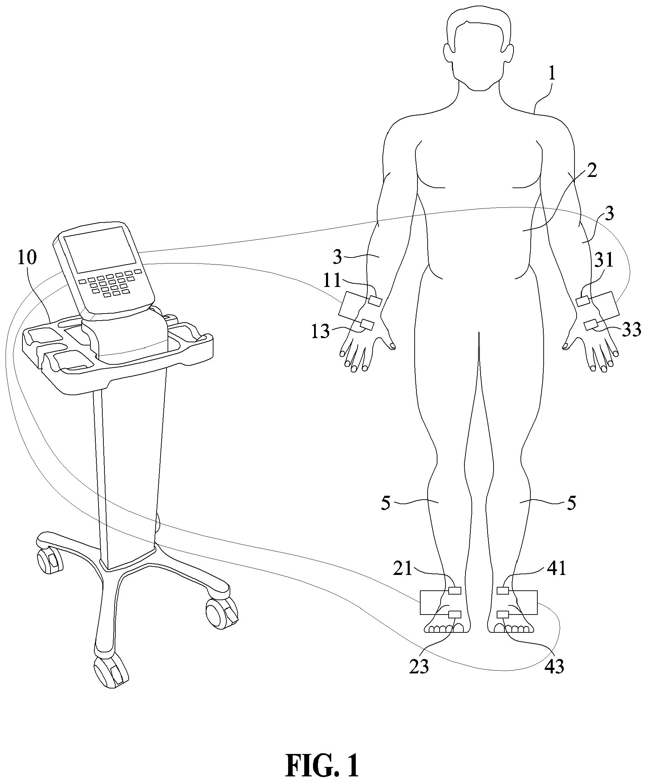

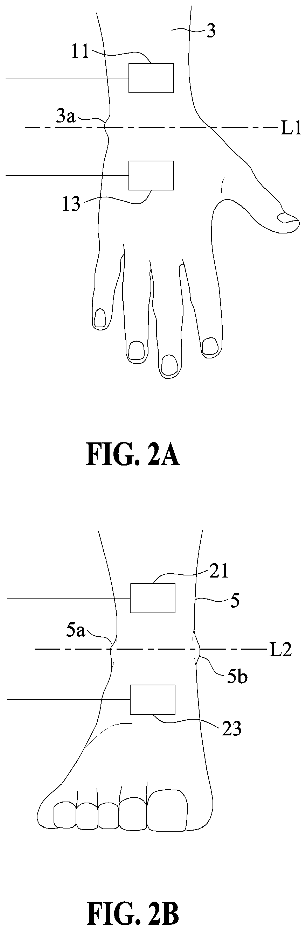

FIG. 1 is a conceptual diagram illustrating body composition measurement according to an embodiment, FIG. 2A is an enlarged view of a part in which an electrode is attached to an arm of a subject in the conceptual diagram of FIG. 1, and FIG. 2B is an enlarged view of a part in which an electrode is attached to a leg of the subject in the conceptual diagram of FIG. 1.

Referring to FIGS. 1, 2A, and 2B, a body composition measuring device 10 may measure and/or analyze the body composition of the subject.

In an embodiment, the body composition measuring device 10 may apply current to at least two points of a body of the subject and may obtain a voltage difference through a built-in voltmeter. In addition, the body composition measuring device 10 may calculate a body impedance value by using the applied current value and the voltage difference and may analyze components of the subject's body or a ratio of the components by using the calculated body impedance value.

In an embodiment, the body composition measuring device 10 may apply current to the subject's body (e.g., limbs or the body). For example, when the subject is a person, a point to which the current is applied may be arms or legs. Alternatively, when the subject is another mammal, the point to which the current is applied may be legs or a body. Hereinafter, the implementations when the subject is a person and the current may be applied to any point of their arms and legs are provided as examples to describe the body composition measuring device 10, but actual implementations are not limited thereto.

In an embodiment, the body composition measuring device 10 may measure an impedance value for a part of the subject's body and may analyze their body composition by using the measured impedance value. For example, a pre-stored formula may be substituted with the impedance value of the body together with another measured value (e.g., a height, a weight, etc.) and the body composition may be calculated and analyzed. In addition, the body composition may also be analyzed with an input of additional input information (e.g., a gender, an age, etc.) to the formula. In an embodiment, the body composition of the subject may be analyzed by preparing a look-up table showing a relationship between the body composition and a measured value and measuring a ratio of the body composition corresponding to the impedance value and the other measured value in the look-up table.

For example, the body composition measuring device 10 may use two current electrodes 11 and 21 and two voltage electrodes 13 and 23 to measure an impedance value (or an electrical resistance value). As illustrated in FIGS. 1, 2A, and 2B, a first current electrode 11 of the body composition measuring device 10 may be attached to a partial region (e.g., a right wrist) of an arm 3 of a subject 1 and a second current electrode 21 of the body composition measuring device 10 may be attached to a partial region (e.g., a right ankle) of a leg 5 of the subject 1. In addition, a first voltage electrode 13 of the body composition measuring device 10 may be attached to the partial region (e.g., the right wrist) of the arm 3 of the subject 1 and a second voltage electrode 23 of the body composition measuring device 10 may be attached to the partial region (e.g., the right ankle) of the leg 5 of the subject 1.

In an embodiment, the body composition measuring device 10 may supply current between the two current electrodes 11 and 21 and may measure a voltage difference between the two voltage electrodes 13 and 23. The body composition measuring device 10 may measure an impedance value by using an amount of the applied current and the voltage difference.

The exemplary description provided above may be the measuring of an impedance value of a body part leading to the right arm 3, the body, and the right leg 5 with the two current electrodes 11 and 21 and the two voltage electrodes 13 and 23 attached to the right wrist and the right ankle, but the actual implementations are not limited to the forgoing implementation.

For example, as illustrated in FIG. 1, a third current electrode 31 of the body composition measuring device 10 may be attached to a partial region (e.g., a left wrist) of the arm 3 of the subject 1, a fourth current electrode 41 of the body composition measuring device 10 may be attached to a partial region (e.g., a left ankle) of the leg 5 of the subject 1, a third voltage electrode 33 of the body composition measuring device 10 may be attached to the partial region (e.g., the left wrist) of the arm 3 of the subject 1, and/or a fourth voltage electrode 43 of the body composition measuring device 10 may be attached to the partial region (e.g., the left ankle) of the leg 5 of the subject 1.

In an embodiment, the body composition measuring device 10 may measure the impedance values of various parts of the subject's body by using at least two of a plurality of current electrodes 11, 21, 31, and 41 and at least two of a plurality of voltage electrodes 13, 23, 33, and 43.

For example, an impedance value leading to the right arm 3, the body, and the left leg 5 may be measured by attaching two current electrodes 11 and 41 and two voltage electrodes 13 and 43 to each of the right wrist and the left ankle. Alternatively, an impedance value leading to the right leg 5 and the left leg 5 may be measured by attaching two current electrodes 21 and 41 and two voltage electrodes 23 and 43 to the right ankle and the left ankle.

In an embodiment, the body composition measuring device 10 may measure a plurality of impedance values, may process the measured impedance values, and may obtain an impedance value for each part: the body, the arm 3, and the leg 5. Alternatively, by varying the combinations of electrodes attached to each part of the body of the subject 1, the body composition measuring device 10 may individually measure the respective impedance values of the body, the arm 3, and the leg 5.

In an embodiment, the body composition measuring device 10 may need to measure the body composition of the subject 1 regularly or repeatedly. For example, in the medical field, by periodically checking a change in body composition, an increase or decrease of water or muscle mass in a patient's body may be figured out. In addition, a doctor may review a new development from body composition analysis results and may estimate a progression or regression state of the patient's disease.

In an embodiment, when the body composition measuring device 10 performs repeated measurements, various electrodes described above may be attached to the subject, may be detached from the subject after a measurement, and may be reattached to the subject when measuring the body composition again. However, since an impedance value of a body may vary depending on a position of an electrode, the electrode may be attached to the position to which the electrode has been attached during a previous measurement. When the electrode is attached to another position that is different from the position to which the electrode has been attached during the previous measurement, the repeatability of the impedance value may decrease. To increase the repeatability, an electrode may be attached to a fixed position every time. To attach the electrode to the fixed position of a subject, an examiner may leave a mark on the body in advance or may attach the electrode to the fixed position of a specific part (e.g., a bony part) of the body, which may be used as a reference mark.

For example, the first current electrode 11 and the first voltage electrode 12 may be attached on the skin respective to preset positions in upward and downward directions of a first reference line L 1 based on the first reference line L 1 generally perpendicular to a length direction of the arm 3 in a position of an ulna head 3a of the subject 1, in the arm 3 of the subject 1.

For example, the second current electrode 21 and the second voltage electrode 22 may be attached on the skin respective to preset positions in upward and downward directions of a second reference line L2, based on the second reference line L2 generally perpendicular to a length direction of the leg 5 in a position of a lateral malleolus 5a or medial malleolus 5b of the subject 1, in the leg 5 of the subject 1.

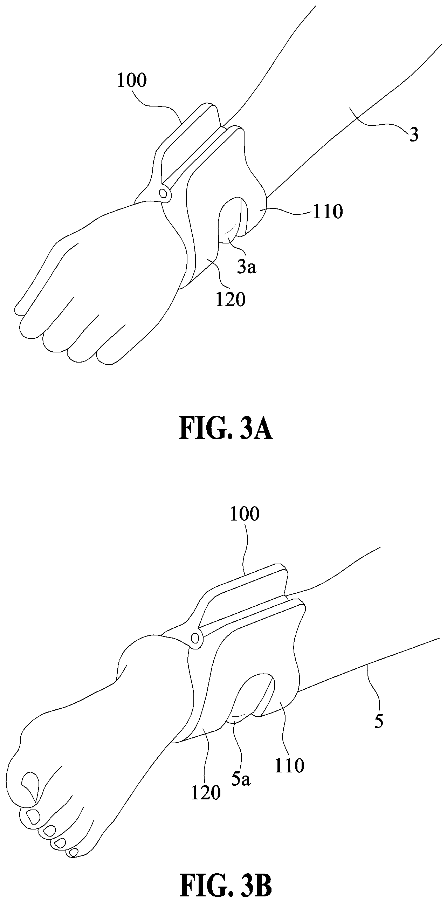

FIG. 3A is a perspective view of a state in which a clamp electrode device is worn on an arm of a subject, according to an embodiment, and FIG. 3B is a perspective view of a state in which the clamp electrode device is worn on a leg of the subject, according to an embodiment.

Referring to FIGS. 3A and 3B, a clamp electrode device 100 may be attached to a body (e.g., the left arm 3 or the left leg 5) of the subject 1.

In an embodiment, the clamp electrode device 100 may be a partial configuration of the body composition measuring device 10 of FIG. 1 or an independent configuration connectable to the body composition measuring device 10. The clamp electrode device 100 may include an electrode inside, may be attached to the subject's body, and may contact the electrode with the subject.

In an embodiment, the clamp electrode device 100 may improve the ease of attachment of the electrode or repeatability. For example, an attachment position may vary depending on the proficiency of a person who attaches the electrode, and the electrode may not be easily attached to or may be easily detached from sweaty skin. However, the clamp electrode device 100 may be mounted to the body at a relatively fixed position, may be easily mounted, may not require a separate adhesive, or may be less likely to fall off due to sweat after being mounted.

In an embodiment, the clamp electrode device 100 may be mounted to a wrist of the arm 3 or an ankle of the leg 5 of the subject 1, and the body composition measuring device 10 may measure the body composition of the subject 1 by using the clamp electrode device 100.

In an embodiment, a first clamp body 110 and second clamp body 120 of the clamp electrode device 100 may be side by side and may be connected to form one device. There may be a gap between the first clamp body 110 and the second clamp body 120 and a specific reference position of the arm 3 or the leg 5 of the subject 1 may be in the gap.

For example, as illustrated in FIG. 3A, when the clamp electrode device 100 is mounted to the arm 3 of the subject 1, the ulna head 3a of the subject 1 may be between the first clamp body 110 and the second clamp body 120. Alternatively, as illustrated in FIG. 3B, when the clamp electrode device 100 is mounted to the leg 5 of the subject 1, the lateral malleolus 5a of the subject 1 may be between the first clamp body 110 and the second clamp body 120.

In an embodiment, the clamp electrode device 100 mounted to the leg 5 of the subject 1 of FIG. 3A and the clamp electrode device 100 mounted to the arm 3 of the subject 1 of FIG. 3B may be substantially the same or similar in structure and size, or the size may vary depending on the thickness of the corresponding body part.

However, the "substantially" herein may be the same level reflecting a tolerance or an error in a general manufacturing process. Alternatively, the "substantially" may be a range including any one range of +/-0.1%, +/-0.5%, +/-1%, +/-3%, +/-5%, +/-7%, +/-10%, +/-15%, and +/-20%, based on the literally same 0%.

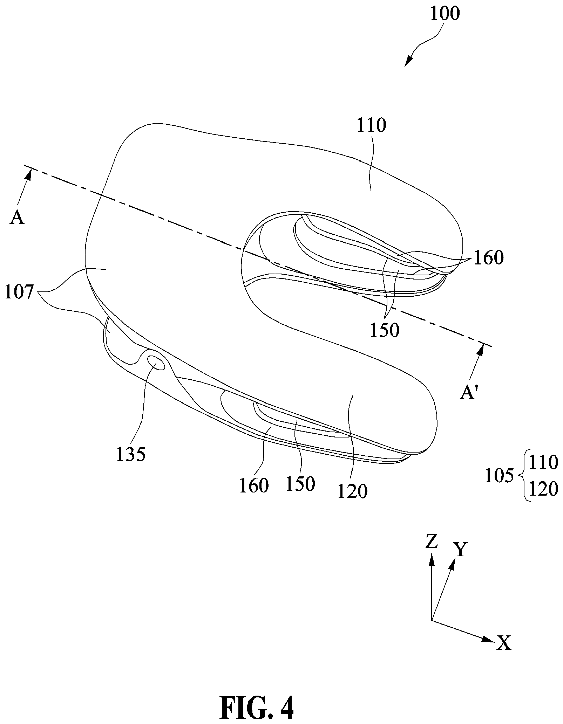

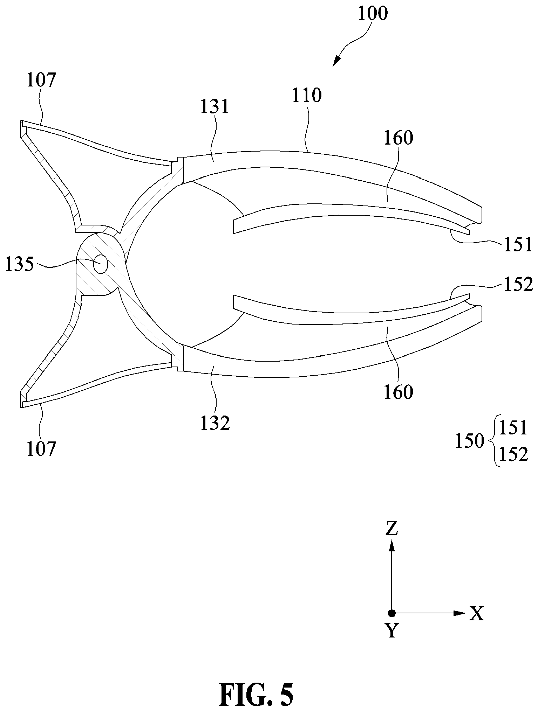

FIG. 4 is a perspective view of a clamp electrode device according to an embodiment, and FIG. 5 is a cross-sectional view of the clamp electrode device according to an embodiment. Specifically, FIG. 5 is a cross-sectional view of the clamp electrode device 100 viewed in the A-A' direction of FIG. 4.

Referring to FIGS. 4 and 5, the clamp electrode device 100 according to an embodiment may include at least one of a clamp body 105, an electrode unit 150, and an elastic member 160.

In an embodiment, the clamp body 105 may form a main body, housing, or body of the clamp electrode device 100. The clamp body 105 may include at least two clamp wings (e.g., a first clamp wing 131 and a second clamp wing 132) for supporting the electrode unit 150 and a hinge unit 135 for supporting and rotating the at least two clamp wings. In addition, the clamp body 105 may include a handle 107 that is in an opposite direction to the electrode unit 150 based on the hinge unit 135. The handle 107 may rotate the clamp body 105.

In an embodiment, the clamp electrode device 100 may be mounted to a user in a method of applying or removing an external force to or from the handle 107. For example, when the external force is applied to the handle 107 by the user or others, tension may be relayed to the hinge unit 135, and the first and second clamp wings 131 and 132 of the clamp body 105 may move respectively in both directions (e.g., +/-Z directions) that are away from each other. When the external force applied to the handle 107 is removed, the first and second clamp wings 131 and 132 of the clamp body 105 may move again to an original position where they are before the external force is applied to the handle 107 by the elasticity of the hinge unit 135, and, as a result, the clamp electrode device 100 may be mounted to the user or restored to an original state.

In an embodiment, the clamp electrode device 100 may include a plurality of clamp bodies 105, and, for example, the clamp bodies 105 may include a first clamp body 110 and a second clamp body 120.

In an embodiment, the first clamp body 110 and the second clamp body 120 may be side by side, and their ends may be connected to each other in one direction (e.g., a -X direction or a handle 107 direction). The first clamp body 110 and the second clamp body 120 may have a shape extending from the ends connected to each other in a direction (e.g., a +X direction) of the other ends that are opposite to the ends. The other ends of the first clamp body 110 and the second clamp body 120 may be spaced from each other and may extend side by side, and there may be a gap between them.

In an embodiment, a specific body part (e.g., the ulna head 3a of FIG. 2A or the lateral and medial malleolus 5a and 5b of FIG. 2B) of the subject may be in the gap between the first clamp body 110 and the second clamp body 120, and the clamp electrode device 100 may be mounted to the subject 1. A protruding body part may function as a reference point or a reference mark, and thus, the clamp electrode device 100 may be mounted to the user in a consistent mounting position. Alternatively, as the protruding body part limits the movement of the clamp electrode device 100, the clamp electrode device 100 may not move in a mounted state and may be prevented from deviation or a change of a position of the electrode unit 150. Although not shown in the drawing, the first clamp body 110 and the second clamp body 120 may be mounted independently, may be separated from each other, or may be independently rotatable at a certain angle. Hereinafter, for ease of description, the examples of the electrode unit 150 and the elastic member 160 of the first clamp body 110 are provided as illustrated in FIG. 5, but the clamp body 105 according to various embodiments herein may be any one or both of the first clamp body 110 and the second clamp body 120.

In an embodiment, the clamp body 105 may include the first clamp wing 131, the second clamp wing 132, and the hinge unit 135. The first clamp wing 131 and the second clamp wing 132 may face each other. The hinge unit 135 may support the first clamp wing 131 and the second clamp wing 132.

For example, an end (e.g., an end in a -X-axis direction) of each of the first clamp wing 131 and the second clamp wing 132 may be supported by the hinge unit 135. The first clamp wing 131 and the second clamp wing 132 may rotate or tilt relative to each other around the hinge unit 135.

In an embodiment, the electrode unit 150 may include a first electrode 151 and a second electrode 152. The first electrode 151 may be on a surface facing the second clamp wing 132 in the first clamp wing 131 and the second electrode 152 may be on a surface facing the first clamp wing 131 in the second clamp wing 132.

As illustrated in FIG. 4, in an embodiment, when the clamp electrode device 100 includes two clamp bodies 105, the clamp electrode device 100 may include two electrode units 150 and a total of four electrodes. Since the two clamp bodies 105 may be spaced apart from each other at a certain distance, one electrode unit 150 in one clamp body 105 may be spaced apart from the other electrode unit 150 in the other clamp body 105 at a certain distance. Although not shown in the drawing, the electrode unit 150 may be connected to an external device (e.g., the body composition measuring device 10 of FIG. 1) through a wire (not shown).

In an embodiment, a plurality of electrodes 151 and 152 of the clamp electrode device 100 may repeatedly contact the skin in respective positions and may repeat measurements. In a repeated measurement process of repeating the mounting and separation of the electrode unit 150, the clamp electrode device 100 may be clamped in a fixed position, the plurality of electrodes 151 and 152 may each contact the skin in the substantially same position as the previous contact position. Thus, despite repeated measurements, the electrodes may each contact the almost same position, and a body composition measurement result with high repeatability may be obtained. As repeatability improves, an examiner may accurately detect a change of body composition over time through periodic, repeated measurement results, may check body condition changes of the subject 1, and may take measures, such as changing treatment methods and/or medications or changing an exercise program for the subj ect 1.

In an embodiment, the elastic member 160 may connect the first electrode 151 to the first clamp wing 131. However, this is just an example, the elastic member 160 according to an embodiment may connect any one of the plurality of electrodes 151 and 152 to any one of a plurality of clamp wings 131 and 132.

In an embodiment, the clamp electrode device 100 may include a plurality of elastic members 160. For example, any one of the plurality of elastic members 160 may connect the second electrode 152 to the second clamp wing 132. The plurality of elastic members 160 may have the substantially same or similar shape and size or may have different shapes and sizes. Hereinafter, for ease of description, the example of the elastic member 160 connecting the first electrode 151 to the first clamp wing 131 is provided, but examples are not limited thereto in actual implementations.

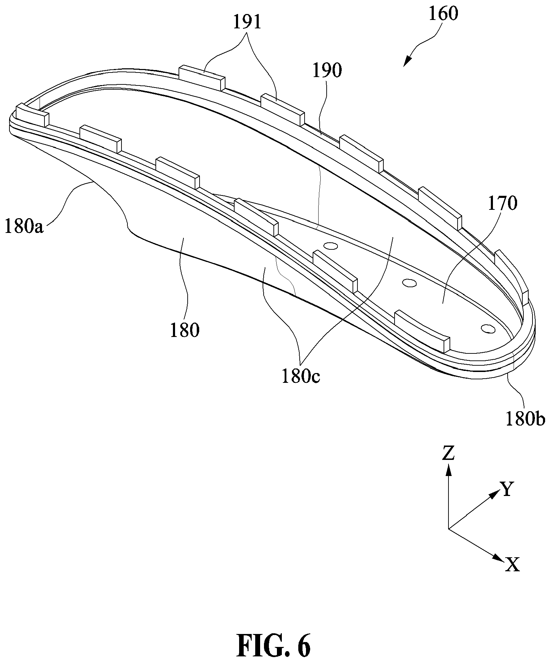

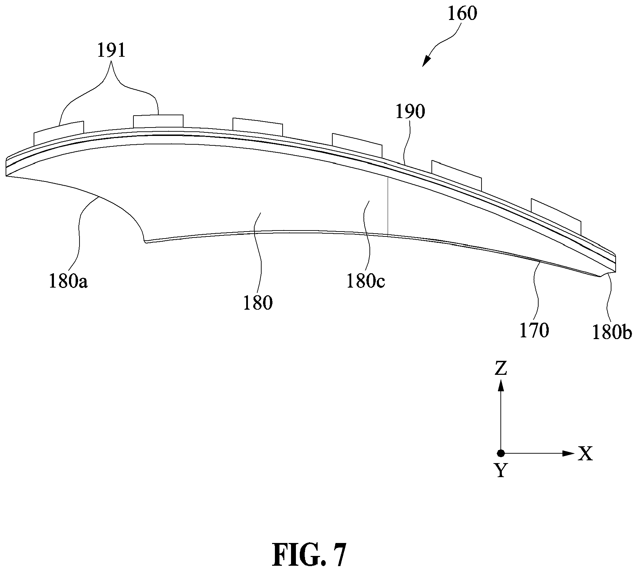

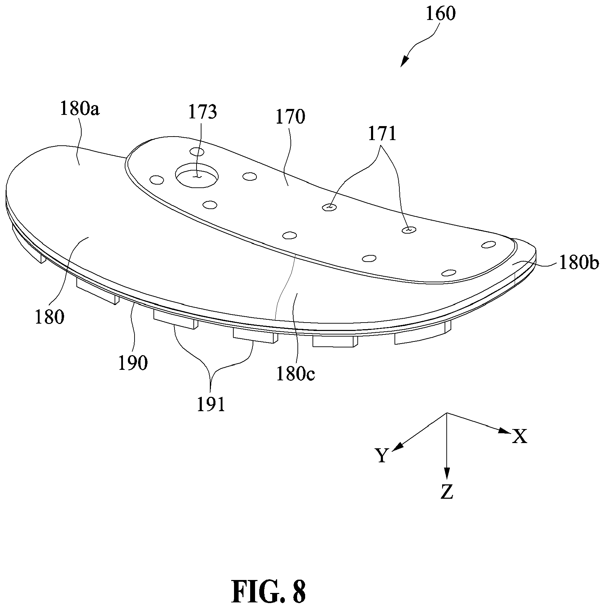

FIG. 6 is a perspective view of an elastic member according to an embodiment, FIG. 7 is a side view of the elastic member according to an embodiment, and FIG. 8 is another perspective view of the elastic member according to an embodiment.

Referring to FIGS. 6, 7, and 8, the elastic member 160 according to an embodiment may include a support surface 170, a sidewall 180, and a fastening region 190.

In an embodiment, the elastic member 160 may support any one (e.g., the first electrode 151) of the electrodes 151 and 152 of the electrode unit 150 and may be coupled to a position (e.g., the first clamp wing 131) of the clamp body 105.

In an embodiment, at least some region of the elastic member 160 may be formed of an elastic material and may include, for example, at least some of rubber and silicone. The elastic member 160 may provide limited flexibility to a position of the first electrode 151 when the clamp electrode device 100 is mounted to the subject 1.

In an embodiment, the support surface 170 of the elastic member 160 may contact in a substantially perpendicular direction to the subject 1, a target to which the clamp electrode device 100 is fastened. Accordingly, the first electrode 151 may be supported by the support surface 170 of the elastic member 160 and may contact in a substantially perpendicular direction to the subject 1. A contact region of the first electrode 151 with the subject 1 may increase through the elastic member 160.

In an embodiment, the first electrode 151 and the support surface 170 of the elastic member 160 may each have a curved shape. Through the curved shape of the first electrode 151, the clamp electrode device 100 may increase a contact area with the subject 1, and the elastic member 160 may improve the bearing capacity of the first electrode 151.

In an embodiment, the support surface 170 may be coupled to the first electrode 151. The support surface 170 may have a substantially flat surface or a curved shape corresponding to the shape of the first electrode 151. The support surface 170 may include a fastening hole 171 and a cable hole 173. The fastening hole 171 may be an opening into which a protruding region (not shown) of the first electrode 151 is inserted and may have a structure for the first electrode 151 to be fastened. The cable hole 173 may be an opening through which a cable (not shown) connected to the first electrode 151 penetrates the elastic member 160.

In an embodiment, the fastening region 190 may be at an end of the sidewall 180 in a direction (e.g., a +Z direction) that is opposite to the support surface 170. The fastening region 190 may be connected to a support body outside of the elastic member 160, for example, the first clamp wing 131, and may be coupled to the clamp electrode device 100.

In an embodiment, the fastening region 190 may lead to a plurality of protrusions 191 spaced apart from one another along the circumference of an end of the sidewall 180. As the plurality of protrusions 191 is inserted into a plurality of insertion grooves (not shown) formed in the first clamp wing 131, the elastic member 160 may be coupled to the first clamp wing 131 without a separate adhesive member.

In an embodiment, the elastic member 160 may be formed of a hollow structure. For example, the elastic member 160 may form an empty space between the support surface 170 and the fastening region 190. Accordingly, the flexibility of the elastic member 160 may increase.

In an embodiment, the sidewall 180 may extend along the circumference of a circumferential surface of the support surface 170 in one direction (e.g., a +Z direction). For example, based on a state in which the first electrode 151 is coupled to the support surface 170, the sidewall 180 may extend to the first clamp wing 131. The sidewall 180 may be formed of a relatively softer elastic material compared to that of another region (e.g., the support surface 170 and/or the fastening region 190) of the elastic member 160. The sidewall 180 may be temporarily transformable by an external force and may provide limited flexibility to the position of the first electrode 151 while the elastic member 160 supports the first electrode 151.

In an embodiment, the sidewall 180 may prevent the first electrode 151 from moving or changing a position while the clamp electrode device 100 is mounted to the subject 1 through various factors, such as a body shape of the subject 1 or the shape and size of a mounted part. In addition, the clamp electrode device 100 may flexibly change its shape suitable for a body curvature of the subject 1 through the elastic member 160.

In an embodiment, the clamp electrode device 100 may be mounted to various users having various body conditions through the elastic member 160, and its versatility may improve. In addition, the elastic member 160 may partially absorb pressure that the subject 1 receives from the first electrode 151 and may reduce or minimize pain felt by the subject 1 from the clamp electrode device 100.

In an embodiment, the sidewall 180 may include a first point 180a and a second point 180b. The first point 180a and the second point 180b may be both ends or both edges, spaced apart from each other or facing each other, of the sidewall 180 or partial regions adjacent to both ends.

For example, the first point 180a, which is one end of the sidewall 180, may be an end closest to the hinge unit 135 or a proximal end towards an inner side of the clamp electrode device 100 when providing a state in which the first point 180a is coupled to the clamp electrode device 100 as an example.

For example, the second point 180b, which is the other end facing the first point 180a, may be an end farthest from the hinge unit 135 or a distal end towards an outer side of the clamp electrode device 100 when providing a state in which the second point 180b is coupled to the clamp electrode device 100 as an example.

In an embodiment, the first point 180a and the second point 180b may have different physical transformable ranges. The first point 180a may be transformed by an external force within a transformable range, and the second point 180b may have a relatively small transformable range compared to the first point 180a.

In an embodiment, the sidewall 180 may provide limited flexibility to the shape of the elastic member 160 through a difference of the transformable ranges of the first point 180a and the second point 180b. This may be implemented in various methods. For example, the first point 180a and the second point 180b may have different shapes and/or lengths or different materials.

In an embodiment, the elastic member 160 may set a transformation degree, transformation direction, and/or transformation limitation of the elastic member 160 through the shape and/or length design of at least a partial region of the sidewall 180. The main transformation degree or transformation direction of the elastic member 160 may be limited by detailed elements of the elastic member 160, such as the extending direction, length, shape, or material of each of the first point 180a and the second point 180b.

For example, as a length in a long-axis direction (e.g., an X-axis direction) from the first point 180a to the second point 180b is longer than a length in a short-axis direction (e.g., a Y-axis direction) perpendicular to the long-axis direction, the main transformation direction of the elastic member 160 may be set to the short-axis direction.

In an embodiment, the limiting of the transformation direction of the elastic member 160 may provide the convenience of measurement for a subj ect and may improve accuracy. For example, while the clamp electrode device 100 is fixed to a body of the subject, as the elastic member 160 provides the main transformation in the short-axis direction, the clamp electrode device 100 may provide flexibility in left and right directions (e.g., the Y-axis direction) corresponding to the short-axis direction, may improve the adhesion of the electrode unit 150, and may reduce the pain of the subj ect.

In an embodiment, the respective lengths of the first point 180a and the second point 180b extending in a vertical direction (e.g., a Z-axis direction) from the support surface 170 to the fastening region 190 may be different, and the transformation ranges of the first point 180a and the second point 180b may be different. Since the first point 180a has a relatively longer length than that of the second point 180b, the first point 180a may have a wide range of transformation by an external force in the vertical direction (e.g., the Z-axis direction) and/or a horizontal direction (e.g., an X-Y plane direction) and may be readily transformed. Since the second point 180b has a relatively shorter length than that of the first point 180a, the second point 180b may have a narrow range of transformation by an external force in the vertical direction and/or the horizontal direction or may not be readily transformed.

In an embodiment, as the transformation ranges of the first point 180a and the second point 180b are different, the elastic member 160 may limit a flexible range of the first electrode 151. For example, in the clamp electrode device 100, a region corresponding to the first point 180a in the first electrode 151 may have a relatively flexible position and a region corresponding to the second point 180b in the first electrode 151 may have a relatively fixed position. Since the region corresponding to the first point 180a is the inner side of the clamp electrode device 100, the elastic member 160 may be transformed variously depending on the body condition of the subject 1 and may improve the repeatability and versatility of the clamp electrode device 100 and the convenience of the subject 1.

In an embodiment, the elastic member 160 may improve a poor contact problem of the electrode unit 150 and/or a pain problem of a subject due to overpressure. When the elastic member 160 is applied to each of the first clamp body 110 and the second clamp body 120, the circumference (e.g., the circumference of the arm 3 or the circumference of the leg 5) of a part of the subject 1 with which the first clamp body 110 contacts and the circumference of a part of the subject 1 with which the second clamp body 120 contacts may be different.

For example, while the electrode unit 150 of the first clamp body 110 contacts with the body, the electrode unit 150 of the second clamp body 120 may not properly contact the body, may partially contact the body, or may not contact the body. The elastic member 160 may support the electrode unit 150 to protrude from the clamp body 105 at a certain distance or may support the electrode unit 150 to be movable in the vertical direction, and thus, the electrode unit 150 may closely contact a contact part regardless of the circumference of the contact part. The elastic member 160 may prevent a poor contact problem by improving the adhesion of the clamp electrode device 100 to a body and/or may reduce pain felt by the subject 1 by reducing the pressure applied to the subject 1.

In an embodiment, the sidewall 180 may include a side surface 180c. The side surface 180c may be a surface leading from the first point 180a to the second point 180b. The side surface 180c may have a gradually shorter length in the vertical direction as it is closer to the second point 180b from the first point 180a. As described above, when the length in the vertical direction shortens, a transformation range of the sidewall 180 decreases, and thus, the side surface 180c may have a smaller transformable range in the vertical direction and/or the horizontal direction as it is closer to the second point 180b from the first point 180a.

In an embodiment, the elastic member 160 may be manufactured in a double injection method. For example, the sidewall 180 may be formed of a soft elastic material (e.g., low-hardness rubber, silicone, etc.) that may be relatively easily transformed compared to the support surface 170 and/or the fastening region 190. The support surface 170 and/or the fastening region 190 may be formed of a relatively hard material (e.g., polycarbonate, high-hardness rubber, silicone, etc.) compared to the sidewall 180. For example, at least some region of the elastic member 160 may include at least one of nylons (e.g., glass-filled nylon (nylon GF)) including glass fibers and may be formed through insert injection.

Hereinafter, the elastic member 160 according to an embodiment, which is applicable to the elastic member 160 or the clamp electrode device 100 described above, is described, and a repeated description thereof is omitted for ease of description. When implementing the embodiments below, at least some of the configurations of the elastic member 160 or the clamp electrode device 100 described above may be omitted, replaced, added, or changed.

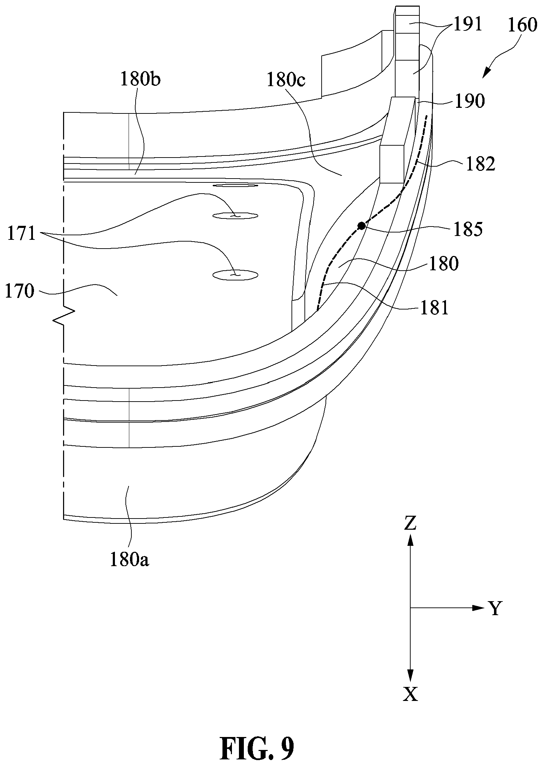

FIG. 9 is an enlarged view of the elastic member 160 according to an embodiment.

Referring to FIG. 9, the sidewall 180 of the elastic member 160 according to an embodiment may include a first curvature region 181 and a second curvature region 182.

In an embodiment, the first curvature region 181 and the second curvature region 182 may be formed on the side surface 180c of the sidewall 180, for example, in a region adj acent to the second point 180b rather than the first point 180a, but examples are not limited thereto. The first curvature region 181 and the second curvature region 182 may be formed on at least some of the first point 180a, second point 180b, and side surface 180c of the sidewall 180. The first curvature region 181 and the second curvature region 182 may structurally limit a transformation range of the elastic member 160 that is soft.

In FIG. 9, for ease of description, an extending direction (or a trend of the extending direction) of the side surface 180c is marked as a virtual dotted line. An inflection region 185, which is a point where the extending direction of the sidewall 180 changes, may be between the first curvature region 181 and the second curvature region 182. The inflection region 185 may extend to at least some region along a direction (e.g., an X-Y plane direction and/or a Z direction) in which the side surface 180c develops.

In an embodiment, the first curvature region 181 may curve and extend in a direction (or a direction toward an outer side of the elastic member 160) away from the inner center of the elastic member 160 from the support surface 170. The second curvature 182 may curve and extend in a direction (or a direction toward the center of the elastic member 160) adjacent to the inner center of the elastic member 160 from the first curvature region 181.

In an embodiment, the first curvature region 181 and the second curvature region 182 may reduce or prevent the decentering of the elastic member 160.

For example, while the clamp electrode device 100 is mounted to a body of a subject, the side surface 180c including the first curvature region 181 and the second curvature region 182 may prevent pressure concentration on the elastic member 160 in a certain direction or in a certain position. In addition, the side surface 180c including the first curvature region 181 and the second curvature region 182 may reduce pain or prevent the subject from feeling pain from decentering or the electrode unit 150 from moving or deviating during the measurement of body composition.

In an embodiment, the first curvature region 181 and the second curvature region 182 may structurally reduce a transformation rate per unit pressure of the sidewall 180. For example, the sidewall 180 including the first curvature region 181 and the second curvature region 182, compared to the sidewall 180 in a straight-line structure or the sidewall 180 curving in one direction, may be relatively limited in transforming in a vertical direction (e.g., a Z-axis direction) and/or in a horizontal direction (e.g., the X-Y plane direction or a Y-axis direction). Accordingly, the sidewall 180 including the first curvature region 181 and the second curvature region 182 may have a reduced transformation rate with respect to the same external force or may have a reduced vertical or horizontal transformable range.

In an embodiment, the transformation conditions and transformation ranges of the sidewall 180 of the elastic member 160 may be adjusted through various designs of the extending directions, lengths, or shapes of the sidewall 180.

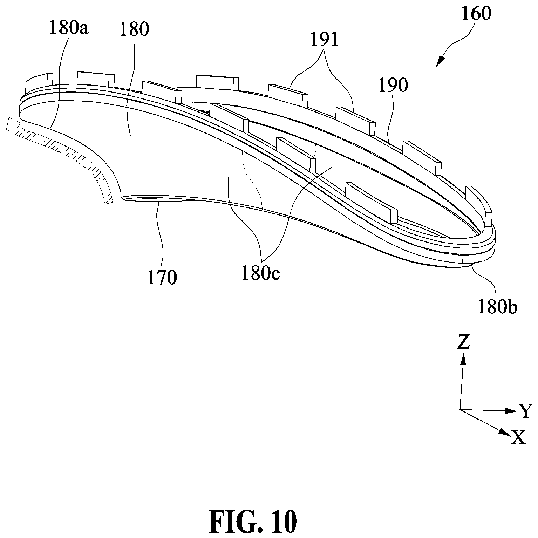

FIG. 10 is another perspective view of the elastic member 160 according to an embodiment.

Referring to FIG. 10, the first point 180a of the elastic member 160 according to an embodiment may have a curved and extended shape.

In an embodiment, the first point 180a may extend in a horizontal direction (e.g., an X-Y plane direction) as well as in a vertical direction (e.g., a Z-axis direction) from the support surface 170. For example, the first point 180a may be gradually away from the second point 180b while extending from the support surface 170. As the first point 180a curves and extends in a direction (e.g., a -X direction) away from the second point 180b, the transformation and restoration of the first point 180a in the vertical direction get smoother and easier, and a transformation range of the first point 180a may increase.

In an embodiment, the first point 180a may curve at a certain curvature and may extend. The first point 180a may be contracted smoothly and transformed in the vertical direction by the curved and extended structure. The curved shape of the first point 180a may reduce pressure on the skin of the subject 1 in a process that the first electrode 151 sits on the body of the subject 1 and may improve the mounting convenience of the subject 1.

In an embodiment, compared to the first point 180a, the second point 180b may relatively extend only in the vertical direction or may have a substantially short extending length. Accordingly, a transformation range of the second point 180b decreases, and thus, the elastic member 160 may substantially fix an outer end of the first electrode 151 to the first clamp wing 131.

In various embodiments herein, by variously designing the extending directions and extending lengths of the first point 180a and the second point 180b, the elastic member 160 may variously set a movement range of the first electrode 151 and a mounting experience of the subject 1 and may improve the versatility and use experience of the clamp electrode device 100.

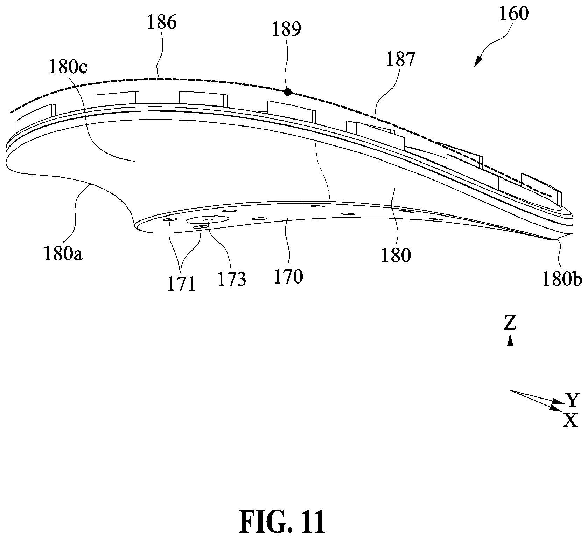

FIG. 11 is another perspective view of the elastic member 160 according to an embodiment.

Referring to FIG. 11, the side surface 180c of the elastic member 160 according to an embodiment may include an inflection point 189.

In an embodiment, the side surface 180c may have a gradually shorter length (or height) in a vertical direction (e.g., a Z-axis direction) as it is closer to the second point 180b from the first point 180a. A reduction rate, which is the reduction rate of the length of the side surface 180c, may be constant or may vary in at least some regions.

In FIG. 11, for ease of description, a length change (or a height change) of the side surface 180c is marked as a virtual dotted line. The inflection point 189 may be a point where the reduction rate of the length of the side surface 180c decreases. For example, the side surface 180c close to the first point 180a based on the inflection point 189 may be a first region 186 and the side surface 180c close to the second point 180b based on the inflection point 189 may be a second region 187.

In an embodiment, compared to the first region 186, the second region 187 may have a low reduction rate of the length of the side surface 180c. Alternatively, the length of the side surface 180c may relatively sharply decrease in the first region 186 and the length of the side surface 180c may relatively moderately decrease in the second region 187.

In an embodiment, a transformation range of the first region 186 and the first point 180a adjacent to the first region 186 may increase and a transformation range of the second region 187 and the second point 180b adjacent to the second region 187 may decrease. Since the first region 186 goes through relatively many changes in the length of the side surface 180c, transformation may be easy, and the transformation range may be large. Since the second region 187 goes through relatively small changes in the length of the side surface 180c, transformation may not be easy, and the transformation range may be small.

In various embodiments herein, by variously designing the reduction rates or reduction trends of the length of the side surface 180c adjacent to the first point 180a and the second point 180b, the elastic member 160 may variously set a movement range of the first electrode 151 and a mounting experience of the subject 1 and may improve the versatility and use experience of the clamp electrode device 100.

In an embodiment, referring to FIG. 11, at least a portion of the support surface 170 and/or the first clamp wing 131 may curve at a certain curvature. For example, considering the structure of the clamp electrode device 100 of FIG. 5 to which the elastic member 160 is mounted, at least some of the first clamp wing 131 and the first electrode 151 may have a curved shape at a certain curvature.

In an embodiment, the fastening region 190 and/or the first clamp wing 131 may extend in a direction (e.g., a +X direction) from the first point 180a to the second point 180b and may gradually curve in a direction (e.g., a -Z direction) to the second clamp wing 132. As the first clamp wing 131 curves, the clamp body 105 may be stably mounted to the body of the subject 1, reflecting a body curvature of the subject 1.

In an embodiment, the support surface 170 and/or the first electrode 151 may extend in a direction (e.g., a +X direction) from the first point 180a to the second point 180b and may curve in a certain direction (e.g., a +/-Z direction). As the support surface 170 and the first electrode 151 curve, the electrode unit 150, by reflecting a body curvature of the subject 1, may stably and closely contact the body of the subject 1.

In an embodiment, based on a state (e.g., the state of FIG. 5) before an external force is applied to the hinge unit 135, the first electrode 151 may extend in a direction from the first point 180a to the second point 180b and may have a curved shape such that the first electrode 151 is away from and closer again to the second electrode 152. Alternatively, the first electrode 151 may have a round shape in which a center direction protrudes upward. When considering the structural shape of a part (e.g., an arm or a leg) to which the clamp electrode device 100 is mounted, the first electrode 151, by having the round structure, may stably and smoothly wrap the body of the subject 1 and may contact the body of the subject 1.

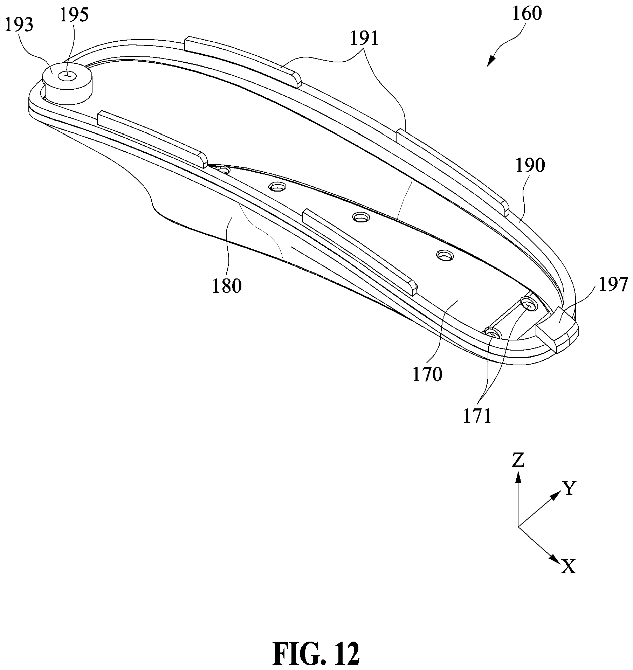

FIG. 12 is another perspective view of the elastic member 160 according to an embodiment.

Referring to FIG. 12, the fastening region 190 of the elastic member 160 according to an embodiment may further include at least some of a fastening member 193 and an insertion member 197.

In an embodiment, the fastening region 190 may include a plurality of protrusions 191 in both side directions (e.g., a +/-Y direction) of the sidewall 180, and the fastening region 190 may further include at least some of the fastening member 193 and the insertion member 197 in both directions (e.g., a +/-X direction).

In an embodiment, the fastening member 193 may be formed at an end of the sidewall 180, for example, an end of the first point 180a. The fastening member 193 may include a through hole 195 formed in the center. The through hole 195 may be formed through in a direction (e.g., a +Z direction) in which the sidewall 180 extends from the support surface 170. The through hole 195 may be inserted into a protruding structure (not shown) of the first clamp wing 131 and may support the coupling of the elastic member 160 to the first clamp wing 131.

In an embodiment, the insertion member 197 may be formed at the other end of the sidewall 180, for example, an end of the second point 180b. The insertion member 197 may have a shape protruding in a direction (e.g., an X-axis direction) horizontal to a direction in which the sidewall 180 extends from the support surface 170. The insertion member 197 may be inserted into an insertion groove (not shown) of the first clamp wing 131 and may support the elastic member 160 and the first clamp wing 131.

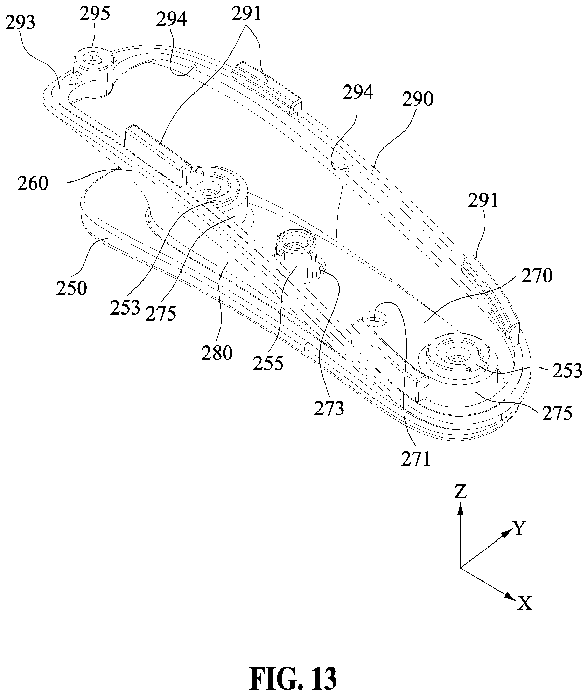

FIG. 13 is a perspective view of a partial configuration of the clamp electrode device according to an embodiment.

Referring to FIG. 13, the clamp electrode device (e.g., the clamp electrode device 100 of FIGS. 3A, 3B, 4, and 5) according to an embodiment may include at least some of an electrode unit 250 (e.g., the electrode unit 150 of FIGS. 4 and 5), an elastic member 260 (e.g., the elastic member 160 of FIGS. 4 to 12), and a fastening member 293 (e.g., the fastening member 193 of FIG. 12).

Hereinafter, the description provided above is not repeated, and it is obvious that a partial configuration or structure of the elastic member 260 and the clamp electrode device 100 including the elastic member 260 may be replaced, added, or omitted within a scope easily understandable by one of ordinary skill in the art with reference to the following drawings and descriptions. In addition, at least one component or feature of the embodiments described above may be coupled to the elastic member 260 and the clamp electrode device 100 including the elastic member 260 unless this is technically and clearly infeasible.

In an embodiment, the elastic member 260 may further include the fastening member 293. The plurality of protrusions 291 may be formed in the fastening member 293. As the plurality of protrusions 291 is inserted into a plurality of insertion grooves (not shown) formed in a clamp wing (e.g., the first clamp wing 131 or the second clamp wing 132), the elastic member 260 may be coupled to the first clamp wing 131 without a separate adhesive member.

In an embodiment, the fastening member 293 may include the through hole 295. The through hole 295 may be formed through in a direction (e.g., a +Z direction) in which a sidewall 280 extends from a support surface 270 of the elastic member 260. The through hole 295 may be inserted into a protruding structure (not shown) of the clamp wing and may support the coupling of the elastic member 260 to the clamp wing.

In an embodiment, the fastening member 293 may be coupled to a fastening region 290 of the elastic member 260. For example, a plurality of fastening protrusions 294 may be formed in the fastening region 290 and may be inserted into the fastening member 293 to support the fastening member 293.

In an embodiment, the fastening member 293 may be a separate structure from the elastic member 260. For example, the fastening member 293 may be one component of the clamp electrode device 100. The fastening member 293 may be between the fastening region 290 and the clamp wing and may assist in connecting the elastic member 260 to the clamp wing.

In an embodiment, the fastening member 293 may have different physical properties from those of the elastic member 260. For example, the elastic member 260 may be formed of a flexible material with an elastic force and the fastening member 293 may be formed of a material with relatively high rigidity. Through the fastening member 293, the elastic member 260 may provide fastening stability with the clamp electrode device 100. In an embodiment, the support surface 270 may be coupled to the electrode unit 250. The support surface 270 may have a substantially flat surface or a curved shape correspondingly to the shape of the electrode unit 250. The support surface 270 may include a first hole 271 and a second hole 273. The first hole 271 may be a structure for the electrode unit 250 to be fastened. A protruding region 255 of the electrode unit 250 may be through the second hole 273. The protruding region 255 of the electrode unit 250 may be connected to a cable (not shown).

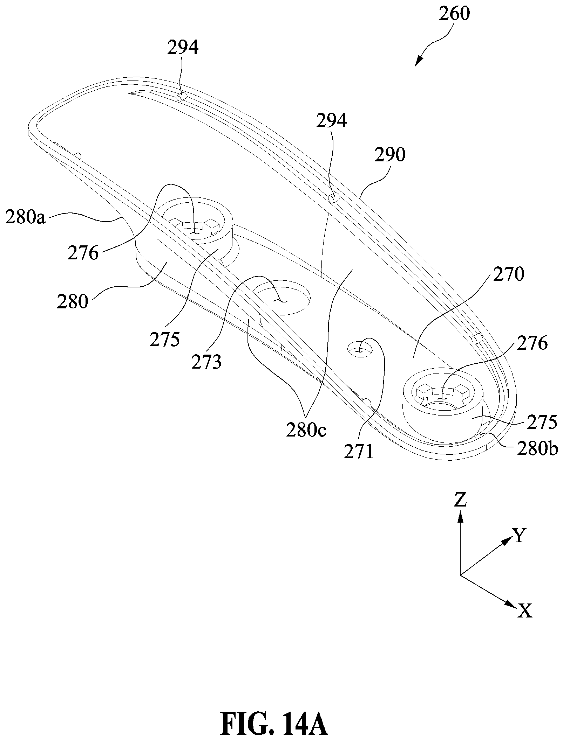

In an embodiment, the support surface 270 may further include a coupling column 275. The coupling column 275 may include an opening (e.g., a coupling opening 276 of FIG. 14A) inside. The coupling column 275 may protrude from the support surface 270. The coupling column 275 may accommodate a coupling member 253 inside and may support the coupling member 253.

In an embodiment, a fastening member (not shown), such as a screw or a bolt, may be fixed to the coupling member 253. The fastening member (now shown) may fix the electrode unit 250 and the elastic member 260.

In an embodiment, the coupling member 253 may reinforce the stiffness of the elastic member 260. When directly coupling the elastic member 260 to the electrode unit 250, the elasticity of the elastic member 260 may reduce a coupling force, may damage a coupled portion, or may release the coupling in use. The coupling member 253 may be formed of a material with relatively high rigidity compared to that of the elastic member 260. Through the coupling member 253, the elastic member 260 may provide coupling stability with the electrode unit 250.

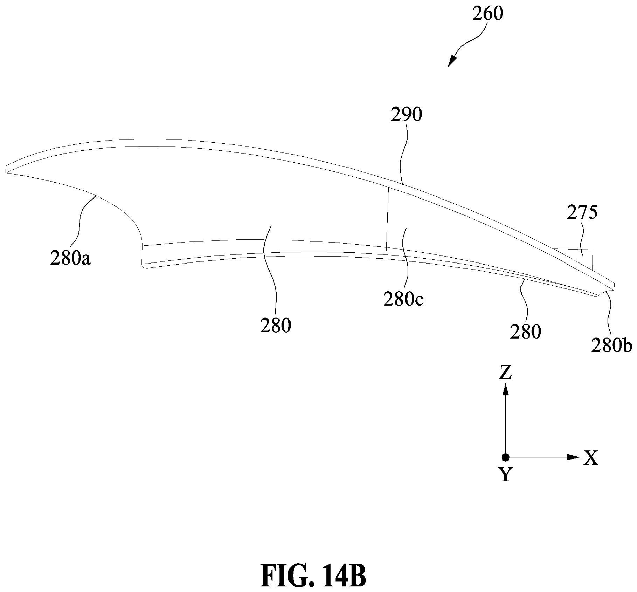

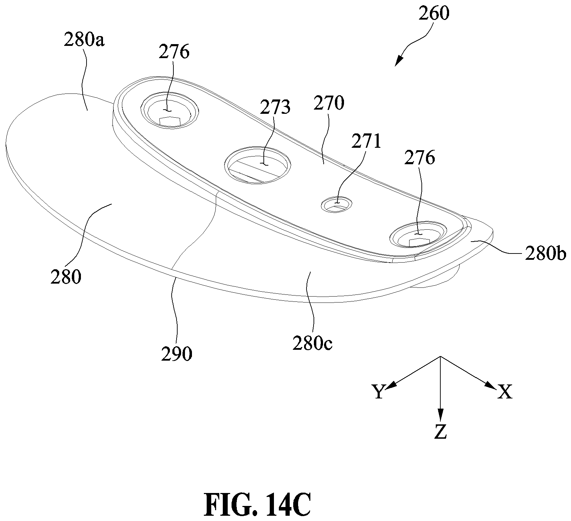

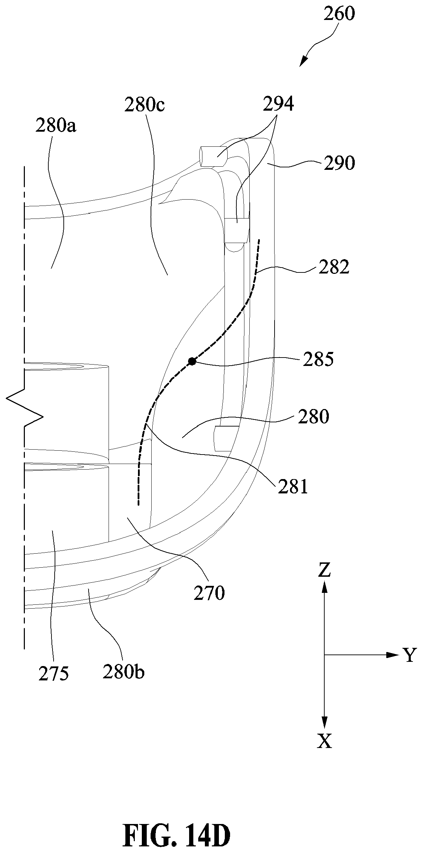

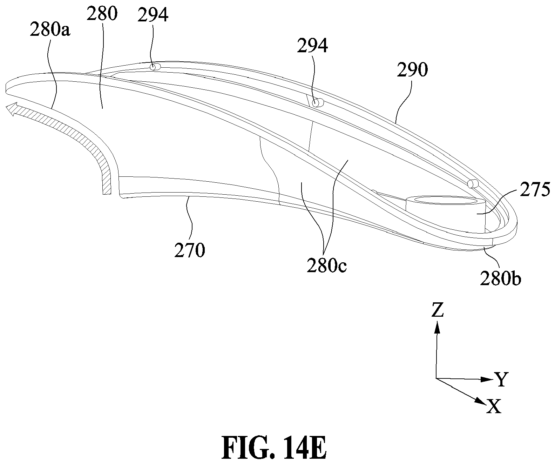

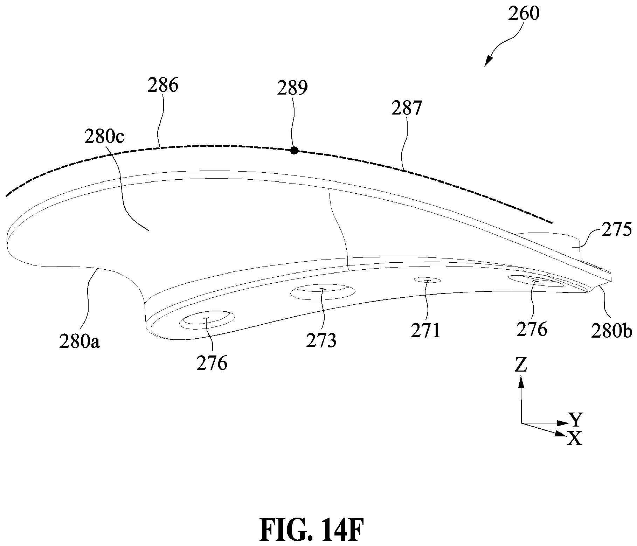

FIG. 14A is a perspective view of the elastic member 260 according to an embodiment, FIG. 14B is a side view of the elastic member 260 according to an embodiment, FIG. 14C is another perspective view of the elastic member 260 according to an embodiment, FIG. 14D is an enlarged view of the elastic member 260 according to an embodiment, FIG. 14E is another perspective view of the elastic member 260 according to an embodiment, and FIG. 14F is another perspective view of the elastic member 260 according to an embodiment.

Referring to FIGS. 14A, 14B, 14C, 14D, 14E, and 14F, the elastic member 260 according to an embodiment may include the support surface 270, the sidewall 280, and the fastening region 290.

In an embodiment, the elastic member 260 may support an electrode unit (e.g., the electrode unit 250 of FIG. 13) and may be coupled to a position (e.g., the first clamp wing 131) of a clamp body (e.g., the clamp body 205 of FIGS. 4 and 5).

In an embodiment, at least some region of the elastic member 260 may be formed of an elastic material and may include, for example, at least some of rubber and silicone. The elastic member 260 may provide limited flexibility to a position of the electrode unit 250 when the clamp electrode device 100 is mounted to the subject 1.

In an embodiment, the fastening region 290 may be at an end of the sidewall 280 in a direction (e.g., a +Z direction) that is opposite to the support surface 270. The sidewall 280 may extend along the circumference of a circumferential surface of the support surface 270 in one direction (e.g., the +Z direction).

For example, based on a state in which the electrode unit 250 is coupled to the support surface 270, the sidewall 280 may extend to the first clamp wing 131. The sidewall 280 may be formed of a relatively softer elastic material compared to that of another region (e.g., the support surface 270 and/or the fastening region 290) of the elastic member 260. The sidewall 280 may be temporarily transformable by an external force and may provide limited flexibility to the position of the electrode unit 250 while the elastic member 260 supports the electrode unit 250.

In an embodiment, the sidewall 280 may prevent the electrode unit 250 from moving or changing a position while the clamp electrode device 100 is mounted to the subject 1 through various factors, such as a body shape of the subject 1 or the shape and size of a mounted part. In addition, the clamp electrode device 100 may flexibly change its shape suitable for a body curvature of the subject 1 through the elastic member 260.

In an embodiment, the clamp electrode device 100 may be mounted to various users having various body conditions through the elastic member 260, and its versatility may improve. In addition, the elastic member 260 may partially absorb pressure that the subject 1 receives from the electrode unit 250 and may reduce or minimize pain felt by the subject 1 from the clamp electrode device 100.

In an embodiment, the sidewall 280 may include a first point 280a and a second point 280b. The first point 280a and the second point 280b may be both ends or both edges, spaced apart from each other or facing each other, of the sidewall 280 or partial regions adjacent to both ends.

For example, the first point 280a, which is one end of the sidewall 280, may be an end closest to a hinge unit 235 or a proximal end towards an inner side of the clamp electrode device 100 when providing a state in which the first point 280a is coupled to the clamp electrode device 100 as an example.

For example, the second point 280b, which is the other end facing the first point 280a, may be an end farthest from the hinge unit 235 or a distal end towards an outer side of the clamp electrode device 100 when providing a state in which the second point 280b is coupled to the clamp electrode device 100 as an example.

In an embodiment, the first point 280a and the second point 280b may have different physical transformable ranges. The first point 280a may be transformed by an external force within a transformable range, and the second point 280b may have a relatively small transformable range compared to the first point 280a.

In an embodiment, the sidewall 280 may provide limited flexibility to the shape of the elastic member 260 through a difference of the transformable ranges of the first point 280a and the second point 280b. This may be implemented in various methods. For example, the first point 280a and the second point 280b may have different shapes and/or lengths or different materials.

In an embodiment, the elastic member 260 may set a transformation degree, transformation direction, and/or transformation limitation of the elastic member 260 through the shape and/or length design of at least a partial region of the sidewall 280. The main transformation degree or transformation direction of the elastic member 260 may be limited by detailed elements of the elastic member 260, such as the extending direction, length, shape, or material of each of the first point 280a and the second point 280b.

For example, as a length in a long-axis direction (e.g., an X-axis direction) from the first point 280a to the second point 280b is longer than a length in a short-axis direction (e.g., a Y-axis direction) perpendicular to the long-axis direction, the main transformation direction of the elastic member 260 may be set to the short-axis direction.

In an embodiment, the limiting of the transformation direction of the elastic member 260 may provide the convenience of measurement for a subj ect and may improve accuracy. For example, while the clamp electrode device 100 is fixed to a body of the subject, as the elastic member 260 provides the main transformation in the short-axis direction, the clamp electrode device 100 may provide flexibility in left and right directions (e.g., the Y-axis direction) corresponding to the short-axis direction, may improve the adhesion of the electrode unit 250, and may reduce the pain of the subj ect.

In an embodiment, the respective lengths of the first point 280a and the second point 280b extending in a vertical direction (e.g., a Z-axis direction) from the support surface 270 to the fastening region 290 may be different, and the transformation ranges of the first point 280a and the second point 280b may be different. Since the first point 280a has a relatively longer length than that of the second point 280b, the first point 280a may have a wide range of transformation by an external force in the vertical direction (e.g., the Z-axis direction) and/or a horizontal direction (e.g., an X-Y plane direction) and may be readily transformed. Since the second point 280b has a relatively shorter length than that of the first point 280a, the second point 280b may have a narrow range of transformation by an external force in the vertical direction and/or the horizontal direction or may not be readily transformed.

In an embodiment, as the transformation ranges of the first point 280a and the second point 280b are different, the elastic member 260 may limit a flexible range of the electrode unit 250. For example, in the clamp electrode device 100, a region corresponding to the first point 280a in the electrode unit 250 may have a relatively flexible position and a region corresponding to the second point 280b in the electrode unit 250 may have a relatively fixed position. Since the region corresponding to the first point 280a is the inner side of the clamp electrode device 100, the elastic member 260 may be transformed variously depending on the body condition of the subject 1 and may improve the repeatability and versatility of the clamp electrode device 100 and the convenience of the subj ect 1.

In an embodiment, the elastic member 260 may improve a poor contact problem of the electrode unit 250 and/or a pain problem of a subject due to overpressure. When the elastic member 260 is applied to each of the first clamp body 210 and the second clamp body 220, the circumference (e.g., the circumference of the arm 3 or the circumference of the leg 5) of a part of the subject 1 with which the first clamp body 210 contacts and the circumference of a part of the subject 1 with which the second clamp body 220 contacts may be different.

For example, while the electrode unit 250 of the first clamp body 210 contacts with the body, the electrode unit 250 of the second clamp body 220 may not properly contact the body, may partially contact the body, or may not contact the body. The elastic member 260 may support the electrode unit 250 to protrude from the clamp body 205 at a certain distance or may support the electrode unit 250 to be movable in the vertical direction, and thus, the electrode unit 250 may closely contact a contact part regardless of the circumference of the contact part. The elastic member 260 may prevent a poor contact problem by improving the adhesion of the clamp electrode device 100 to a body and/or may reduce pain felt by the subject 1 by reducing the pressure applied to the subject 1.

In an embodiment, the sidewall 280 may include a side surface 280c. The side surface 280c may be a surface leading from the first point 280a to the second point 280b. The side surface 280c may have a gradually shorter length in the vertical direction as it is closer to the second point 280b from the first point 280a. As described above, when the length in the vertical direction shortens, a transformation range of the sidewall 280 decreases, and thus, the side surface 280c may have a smaller transformable range in the vertical direction and/or the horizontal direction as it is closer to the second point 280b from the first point 280a.

In an embodiment, the elastic member 260 may be manufactured in a double injection method. For example, the sidewall 280 may be formed of a soft elastic material (e.g., low-hardness rubber, silicone, etc.) that may be relatively easily transformed compared to the support surface 270 and/or the fastening region 290. The support surface 270 and/or the fastening region 290 may be formed of a relatively hard material (e.g., polycarbonate, high-hardness rubber, silicone, etc.) compared to the sidewall 280. For example, at least some region of the elastic member 260 may include at least one of nylons (e.g., nylon GF) including glass fibers and may be formed through insert injection.

In an embodiment, the sidewall 280 of the elastic member 260 may include the first curvature region 281 and the second curvature region 282. The first curvature region 281 and the second curvature region 282 may be formed on the side surface 280c of the sidewall 280, for example, in a region adjacent to the second point 280b rather than the first point 280a, but examples are not limited thereto. The first curvature region 281 and the second curvature region 282 may be formed at least some of the first point 280a, second point 280b, and side surface 280c of the sidewall 280. The first curvature region 281 and the second curvature region 282 may structurally limit a transformation range of the elastic member 260 that is soft.

In FIG. 9, for ease of description, an extending direction (or a trend of the extending direction) of the side surface 280c is marked as a virtual dotted line. An inflection region 285, which is a point where the extending direction of the sidewall 280 changes, may be between the first curvature region 281 and the second curvature region 282. The inflection region 285 may extend to at least some region along a direction (e.g., an X-Y plane direction and/or a Z direction) in which the side surface 280c develops.

In an embodiment, the first curvature region 281 may curve and extend in a direction (or a direction toward an outer side of the elastic member 260) away from the inner center of the elastic member 260 from the support surface 270. The second curvature 282 may curve and extend in a direction (or a direction toward the center of the elastic member 260) adjacent to the inner center of the elastic member 260 from the first curvature region 281.

In an embodiment, the first curvature region 281 and the second curvature region 282 may reduce or prevent the decentering of the elastic member 260.

For example, while the clamp electrode device 100 is mounted to a body of a subject, the side surface 280c including the first curvature region 281 and the second curvature region 282 may prevent pressure concentration on the elastic member 260 in a certain direction or in a certain position. In addition, the side surface 280c including the first curvature region 281 and the second curvature region 282 may reduce pain or prevent the subject from feeling pain from decentering or the electrode unit 250 from moving or deviating during the measurement of body composition.

In an embodiment, the first curvature region 281 and the second curvature region 282 may structurally reduce a transformation rate per unit pressure of the sidewall 280. For example, the sidewall 280 including the first curvature region 281 and the second curvature region 282, compared to the sidewall 280 in a straight-line structure or the sidewall 280 curving in one direction, may be relatively limited in transforming in a vertical direction (e.g., a Z-axis direction) and/or in a horizontal direction (e.g., the X-Y plane direction or a Y-axis direction). Accordingly, the sidewall 280 including the first curvature region 281 and the second curvature region 282 may have a reduced transformation rate with respect to the same external force or may have a reduced vertical or horizontal transformable range.

In an embodiment, the transformation conditions and transformation ranges of the sidewall 280 of the elastic member 260 may be adjusted through various designs of the extending directions, lengths, or shapes of the sidewall 280.

In an embodiment, the elastic member 260 may be formed of a hollow structure. For example, the elastic member 260 may form an empty space between the support surface 270 and the fastening region 290. Accordingly, the flexibility of the elastic member 260 may increase.

In an embodiment, the first point 280a of the elastic member 260 may have a curved and extended shape. The first point 280a may extend in a horizontal direction (e.g., an X-Y plane direction) as well as in a vertical direction (e.g., a Z-axis direction) from the support surface 270. For example, the first point 280a may be gradually away from the second point 280b while extending from the support surface 270. As the first point 280a curves and extends in a direction (e.g., a -X direction) away from the second point 280b, the transformation and restoration of the first point 280a in the vertical direction get smoother and easier, and a transformation range of the first point 280a may increase.

In an embodiment, the first point 280a may curve at a certain curvature and may extend. The first point 280a may be contracted smoothly and transformed in the vertical direction by the curved and extended structure. The curved shape of the first point 280a may reduce pressure on the skin of the subject 1 in a process that the electrode unit 250 sits on the body of the subject 1 and may improve the mounting convenience of the subject 1.

In an embodiment, compared to the first point 280a, the second point 280b may relatively extend only in the vertical direction or may have a substantially short extending length. Accordingly, a transformation range of the second point 280b decreases, and thus, the elastic member 260 may substantially fix an outer end of the electrode unit 250 to the first clamp wing 131.

In various embodiments herein, by variously designing the extending directions and extending lengths of the first point 280a and the second point 280b, the elastic member 260 may variously set a movement range of the electrode unit 250 and a mounting experience of the subject 1 and may improve the versatility and use experience of the clamp electrode device 100.

In an embodiment, the side surface 280c of the elastic member 260 may include an inflection point 289. The side surface 280c may have a gradually shorter length (or height) in a vertical direction (e.g., a Z-axis direction) as it is closer to the second point 280b from the first point 280a. A reduction rate, which is the reduction rate of the length of the side surface 280c, may be constant or may vary in at least some regions.

In the drawing, for ease of description, a length change (or a height change) of the side surface 280c is marked as a virtual dotted line. The inflection point 289 may be a point where the reduction rate of the length of the side surface 280c decreases. For example, the side surface 280c close to the first point 280a based on the inflection point 289 may be a first region 286 and the side surface 280c close to the second point 280b based on the inflection point 289 may be a second region 287.

In an embodiment, compared to the first region 286, the second region 287 may have a low reduction rate of the length of the side surface 280c. Alternatively, the length of the side surface 280c may relatively sharply decrease in the first region 286 and the length of the side surface 280c may relatively moderately decrease in the second region 287.

In an embodiment, a transformation range of the first region 286 and the first point 280a adjacent to the first region 286 may increase and a transformation range of the second region 287 and the second point 280b adjacent to the second region 287 may decrease. Since the first region 286 goes through relatively many changes in the length of the side surface 280c, transformation may be easy, and the transformation range may be large. Since the second region 287 goes through relatively small changes in the length of the side surface 280c, transformation may not be easy, and the transformation range may be small.

In various embodiments herein, by variously designing the reduction rates or reduction trends of the length of the side surface 280c adjacent to the first point 280a and the second point 280b, the elastic member 260 may variously set a movement range of the electrode unit 250 and a mounting experience of the subject 1 and may improve the versatility and use experience of the clamp electrode device 100.

In an embodiment, at least a portion of the support surface 270 and/or the first clamp wing 131 may curve at a certain curvature. For example, considering the structure of the clamp electrode device 100 of FIG. 5 to which the elastic member 260 is mounted, at least some of the first clamp wing 131 and the electrode unit 250 may have a curved shape at a certain curvature.

In an embodiment, the fastening region 290 and/or the first clamp wing 131 may extend in a direction (e.g., a +X direction) from the first point 280a to the second point 280b and may gradually curve in a direction (e.g., a -Z direction) to the second clamp wing 132. As the first clamp wing 131 curves, the clamp body 205 may be stably mounted to the body of the subject 1, reflecting a body curvature of the subject 1.

In an embodiment, the support surface 270 and/or the electrode unit 250 may extend in a direction (e.g., a +X direction) from the first point 280a to the second point 280b and may curve in a certain direction (e.g., a +/-Z direction). As the support surface 270 and the electrode unit 250 curve, the electrode unit 250, by reflecting a body curvature of the subject 1, may stably and closely contact the body of the subject 1.

In an embodiment, based on a state (e.g., the state of FIG. 5) before an external force is applied to the hinge unit 235, the electrode unit 250 may extend in a direction from the first point 280a to the second point 280b and may have a curved shape such that the first electrode 151 is away from and closer again to the second electrode 152. Alternatively, the electrode unit 250 may have a round shape in which a center direction protrudes upward. When considering the structural shape of a part (e.g., an arm or a leg) to which the clamp electrode device 100 is mounted, the electrode unit 250, by having the round structure, may stably and smoothly wrap the body of the subject 1 and may contact the body of the subject 1.

The examples described herein may be implemented by using a hardware component, a software component, and/or a combination thereof. A processing device may be implemented using one or more general-purpose or special-purpose computers, such as, for example, a processor, a controller and an arithmetic logic unit (ALU), a digital signal processor (DSP), a microcomputer, a field-programmable gate array (FPGA), a programmable logic unit (PLU), a microprocessor, or any other device capable of responding to and executing instructions in a defined manner. The processing device may run an operating system (OS) and one or more software applications that run on the OS. The processing unit also may access, store, manipulate, process, and generate data in response to execution of the software. For simplicity, the description of a processing unit is used as singular; however, one skilled in the art will appreciate that a processing unit may include multiple processing elements and multiple types of processing elements. For example, the processing unit may include a plurality of processors, or a single processor and a single controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described examples may be recorded in non-transitory computer-readable media including program instructions to implement various operations of the above-described examples. The media may also include, alone or in combination with the program instructions, data files, data structures, and the like. The program instructions recorded on the media may be those specially designed and constructed for the purposes of examples, or they may be of the kind well-known and available to those having skill in the computer software arts. Examples of non-transitory computer-readable media include magnetic media such as hard disks, floppy disks, and magnetic tape; optical media such as CD-ROM discs and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher-level code that may be executed by the computer using an interpreter.

The above-described devices may act as one or more software modules in order to perform the operations of the above-described examples, or vice versa.

As described above, although the examples have been described with reference to the limited drawings, a person skilled in the art may apply various technical modifications and variations based thereon. For example, suitable results may be achieved if the described techniques are performed in a different order and/or if components in a described system, architecture, device, or circuit are combined in a different manner and/or replaced or supplemented by other components or their equivalents.

Therefore, other implementations, other examples, and equivalents to the claims are also within the scope of the following claims.

## Claims

1. A clamp electrode device comprising:
a clamp body comprising a first clamp wing, a second clamp wing facing the first clamp wing, and a hinge unit supporting an end of each of the first clamp wing and the second clamp wing;
an electrode unit comprising a first electrode on a surface facing the second clamp wing in the first clamp wing and a second electrode on a surface facing the first clamp wing in the second clamp wing; and
an elastic member connecting the first electrode to the first clamp wing.

2. The clamp electrode device of claim 1, wherein the elastic member comprises:
a support surface to which the first electrode is coupled;
a sidewall extending to the first clamp wing from the support surface; and
a fastening region that is at an end opposite to the support surface in the sidewall and connected to the first clamp wing.

3. The clamp electrode device of claim 2, wherein the sidewall comprises:
a soft elastic material that is relatively easily transformable compared to the support surface and the fastening region.

4. The clamp electrode device of claim 2, wherein the sidewall comprises:
a first point that is an end closest to the hinge unit and transformable by an external force within a transformable range; and
a second point that is the other end facing the first point and of which a transformable range is less than that of the first point.

5. The clamp electrode device of claim 4, wherein
the second point forms a relatively short extending length from the support surface to the fastening region compared to that of the first point.

6. The clamp electrode device of claim 4, wherein
the first point extends from the support surface and has a curved shape at a certain curvature such that the first point is gradually away from the second point.

7. The clamp electrode device of claim 4, wherein
the sidewall comprises a side surface leading from the first point to the second point, and
the side surface has a length extending from the support surface, which gradually shortens as the side surface is closer to the second point from the first point.

8. The clamp electrode device of claim 7, wherein the side surface comprises:
a first curvature region extending while curving in a direction away from an inner center of the elastic member from the support surface; and
a second curvature region extending while curving in a direction adjacent to the inner center of the elastic member from the first curvature region.

9. The clamp electrode device of claim 7, wherein the side surface comprises:
an inflection point where a reduction rate, or a rate at which the length shortens, decreases while the side surface is closer to the second point from the first point.

10. The clamp electrode device of claim 4, wherein
the first clamp wing extends in a direction from the first point to the second point and has a curved shape gradually closer to the second clamp wing.

11. The clamp electrode device of claim 4, wherein
the support surface has a curved shape extending in a direction from the first point to the second point.

12. The clamp electrode device of claim 4, wherein
the first electrode, based on a state before an external force is applied to the hinge unit, extends in a direction from the first point to the second point and has a curved shape such that the first electrode is away from and closer again to the second electrode.

13. The clamp electrode device of claim 1, wherein the elastic member comprises a hollow structure.

14. An elastic member for a clamp electrode device, the elastic member comprising:
a support surface facing a fastening target of the clamp electrode device;
a sidewall extending from the support surface; and
a fastening region that is at an end opposite to the support surface in the sidewall and coupled to the clamp electrode device.

15. The elastic member of claim 14, wherein the sidewall comprises:
a first point that is an end and is transformable by an external force within a transformable range; and
a second point that is the other end facing the first point and of which a transformable range is less than that of the first point.
